# EUROPÄISCHE PATENTSCHRIFT

(11) **EP 3 787 743 B1**
(45) Veröffentlichungstag und Bekanntmachung des Hinweises auf die Patenterteilung: **27.12.2023**
(21) Anmeldenummer: 19719510.0
(22) Anmeldetag: 24.04.2019
(51) Int. Cl.: A61N 5/10, A61B 90/14, A61B 90/18, A61B 13/00, A61B 6/04, A61B 90/16, A61F 5/37

(54) **VORRICHTUNG ZUR IMMOBILISIERUNG EINES PATIENTENKOPFES**
DEVICE FOR IMMOBILIZING THE HEAD OF A PATIENT
DISPOSITIF D'IMMOBILISATION DE LA TÊTE D'UN PATIENT

(30) Priorität: 04.05.2018 DE 102018110707; 01.08.2018 DE 102018118704
(43) Veröffentlichungstag der Anmeldung: 10.03.2021
(73) Patentinhaber: IT-V Medizintechnik GmbH, 6020 Innsbruck (AT)
(72) Erfinder: VÖLP, Markus, 6020 Innsbruck (AT)
(74) Vertreter: Behr, Wolfgang
(86) Internationale Anmeldenummer: PCT/EP2019/060440
(87) Internationale Veröffentlichungsnummer: WO 2019/211132

(56) Entgegenhaltungen:
- WO-A1-2014/193938
- US-A1- 2017 333 243

## Beschreibung

Die Erfindung betrifft eine Vorrichtung und ein Verfahren zur Immobilisierung des Kopfes eines Patienten in einer Anlage zur Durchführung einer Strahlentherapie.

In einer Vielzahl an medizinischen Diagnose- und Behandlungsverfahren ist es erfordern, den Kopf eines Patienten in einer genauen und reproduzierbaren Position zu fixieren. Ein Beispiel für ein solches Behandlungsverfahren ist eine kraniale Strahlentherapie, bei der ein Hirntumor bestrahlt werden soll. Insbesondere ist es in diesem Fall notwendig, den Kopf des Patienten relativ zu einem schon während der vorgelagerten Tomographie festzulegenden Bezugssystem zu immobilisieren. Bei einer Bestrahlung eines HNO-Karzinoms kann zudem auch eine Fixierung des Kiefers und der Zunge erforderlich sein.

Im Stand der Technik ist es bekannt, für eine derartige Immobilisierung eine thermoplastische Maske zu verwenden, die sich an die Konturen des Patientenkopfes anpasst. Die Maske wird zur Reproduktion der ursprünglichen Position während späteren Behandlungsschritten wieder auf den Patientenkopf gesetzt und in derselben Position am Behandlungsplatz bzw. -tisch fixiert. Beispielhaft kann in diesem Zusammenhang auf die EP 2 476 375 A1 verwiesen werden.

Um eine erhöhte Positioniergenauigkeit der thermoplastischen Maske am Kopf des Patienten zu erreichen und Bewegungen wie Kopfbewegungen und insbesondere Kieferbewegungen bei aufgesetzter Maske weiter zu verringern, wurde in der US 2002/108616 A1 ein Konzept vorgestellt, bei dem ein Mundstück an der thermoplastischen Maske fixiert wird. Dieses Konzept hat aber unter anderem den Nachteil, dass die Atmung des Patienten unter Umständen beeinträchtigt wird und es zu Unwohlsein oder sogar Panik des Patienten kommen kann.

In der WO 2014/193938 A1 wird eine gattungsgemäße Vorrichtung umfassend eine entsprechend der Kontur des Patientenkopfes ausgeformte thermoplastische Maske und ein Mundstück vorgeschlagen, bei welcher der mundbedeckende Abschnitt der noch weichen Maske anhand des Mundstücks in den Mund gedrückt und das Mundstück so an der Maske bzw. im Mund des Patienten fixiert werden soll. Auch diese Lösung ist aber für den Patienten nicht komfortabel.

Aufgabe der Erfindung ist es, ein verbessertes Konzept zur Immobilisierung des Patientenkopfes bereitzustellen, welches die Nachteile des Standes der Technik überwindet.

Vor diesem Hintergrund betrifft die Erfindung eine Vorrichtung zur Immobilisierung des Kopfes eines Patienten in einer Anlage zur Durchführung einer Strahlentherapie, wobei die Vorrichtung eine entsprechend der Kontur des Patientenkopfes ausgeformte thermoplastische Maske und ein Mundstück umfasst, wobei das Mundstück ein Mittelteil aufweist, von dem in orale Richtung ein Schnabel und in aborale Richtung eine Haltestruktur abstehen, und wobei ein Luftkanal vorgesehen ist, der das Mundstück vom mundseitigen Ende des Schnabels bis an die aborale Seite des Mittelteils durchdringt. Erfindungsgemäß ist vorgesehen, dass ein Bereich der Maske entsprechend der Kontur der Haltestruktur ausgeformt ist.

Die Haltestruktur erstreckt sich aus der Auflageebene der Maske auf das Mundstück heraus, um in der Maske abgebildet werden zu können. Durch die korrespondierende Formgebung der Haltestruktur des Mundstücks einerseits und der korrespondierenden Ausformung in der Maske andererseits wird eine Bewegung wie beispielsweise Verschiebung oder Verdrehung des Mundstücks in der Auflageebene der Maske verhindert. Insbesondere kann eine genaue und reproduzierbare Position des Mundstücks im Verhältnis zur Maske festgelegt werden.

Der Schnabel dient einerseits zur Definition des Luftkanals und andererseits zur Fixierung des Mundstücks zwischen den Kiefern des Patienten. Je nachdem, ob ein HNO-Tumor oder ein Hirntumor bestrahlt werden soll, kann der Schnabel etwas breiter sein, um Ober- und Unterkiefer auseinanderzuhalten und die Zunge nach unten zu drücken, oder etwas schmaler sein, um den Komfort zu erhöhen.

Bei der thermoplastischen Maske handelt es sich um einen originär planen Materialbogen, der nach Erwärmung entsprechend der Kontur des Patientenkopfes und der Haltestruktur ausgeformt wird und diese Form nach Abkühlung behält. Das thermoplastische Kunststoffmaterial des Maskenbogens ist so gewählt, dass es nach Erwärmung auf eine Arbeitstemperatur von etwa 50°C bis allenfalls 100°C verformbar wird und nach Abkühlung auf eine Temperatur unterhalb der Arbeitstemperatur, jedenfalls aber nach Abkühlung auf unter 40°C erstarrt. Vorzugsweise ist die Maske siebartig gelocht.

In einer Ausführungsform ist vorgesehen, dass das thermoplastische Maskenmaterial in einem Maskenrahmen eingefasst ist. Der Maskenrahmen kann seinerseits am Behandlungsplatz der Anlage fixiert werden, beispielsweise auf einem Behandlungstisch.

In einer Ausführungsform ist vorgesehen, dass der Maskenrahmen eine U-förmige Gestalt hat und an einer Basisplatte fixiert werden kann, die auf den Behandlungstisch aufgesetzt werden kann oder Teil des Behandlungstisches darstellt. Der Patientenkopf wird dann zwischen Basisplatte und dem aus der Ebene des Maskenrahmens geformten, thermoplastischen Maskenmaterial fixiert. Ein Beispiel für ein derartiges System ist in der EP 2 476 375 A1 offenbart.

Der Mittelteil kann in einer Ausführungsform so geformt sein, dass es als Begrenzung dient und die maximale Eindringtiefe des Schnabels in den Mund des Patienten festgelegen kann. In diesem Zusammenhang kann vorgesehen sein, dass das Mundstück nicht mit der Maske verbunden ist und mit seiner Haltestruktur lose in der entsprechenden Ausformung der Maske sitzt. Es sind in dieser Ausführungsform also keine Fixierungsmittel wie beispielsweise Schrauben oder Rastelemente vorhanden, um das Mundstück mit der Maske zu verbinden. Ebenso wenig ist in dieser Ausführungsform eine materialschlüssige Verbindung wie eine Klebeverbindung oder Schweißverbindung vorgesehen. Die Haltestruktur sitzt lediglich lose in der korrespondierenden Ausformung der Maske und kann senkrecht zur Auflageebene der Maske einfach und im Wesentlichen widerstandsfrei aus dieser Ausformung gezogen werden. Die Ausformung weist keine Hinterschneidungen oder dergleichen auf, um eine Relativbewegung senkrecht zur Auflageebene der Maske zu verhindern oder zu erschweren.

In einer Ausführungsform ist vorgesehen, dass die Haltestruktur wenigstens zwei von dem Mittelteil abstehende Strukturelemente aufweist und/oder dass das oder die Strukturelemente der Haltestruktur eine in der Auflageebene der Maske ausgedehnte Gestalt haben.

Durch diese Gestaltung wird eine Mehrpunktfixierung erreicht, sodass eine Rotation des Mundstücks im Verhältnis zur Maske nicht und ein Verkippen nicht oder nur schwer möglich ist. Die Strukturelemente können beispielsweise punktförmige Stifte oder in der Auflageebene der Maske ausgedehnte Stege umfassen, die vorzugsweise im Wesentlichen senkrecht auf die Auflageebene der Maske, mithin also in aborale Richtung stehen.

In einer Ausführungsform umfassen die Strukturelemente eine in der Achse des Schnabels von dem Mittelteil abstehende Zinne, wobei sich der Luftkanal bis zum aboralen Ende dieser Zinne fortsetzt. Die Zinne kann in einer Ausführungsform zumindest geringfügig alle anderen Strukturelemente der Haltestruktur in aborale Richtung überragen. Durch die Zinne kann die Fixierung der Maske am Mundstück verbessert werden. Ferner kann durch das Abheben der Austrittsöffnung des Luftkanals vom Basisteil im Falle eines Erbrechens des Patienten vermieden werden, dass das Erbrochene wieder zurück in den Luftkanal gelangt.

In einer Ausführungsform umfasst die Haltestruktur wenigstens drei Strukturelemente, nämlich zwei Stege sowie eine derartige Zinne.

In einer Ausführungsform ist vorgesehen, dass in demjenigen Bereich der Maske, der entsprechend der Kontur der Haltestruktur ausgeformt ist, ein Durchbruch eingearbeitet ist. Da die aborale Öffnung des durch das Mundstück führenden Luftkanals an der aboralen Seite des Mittelteils und mithin im Bereich der Haltestrukturen liegt, befindet sich ein derartiger Durchbruch im Bereich der aboralen Öffnung des Luftkanals. Selbst wenn das Maskenmaterial an sich bereits siebartig gelocht ist, erleichtert das Vorhandensein eines im Gegensatz zu den kleinen regelmäßigen Lochstanzungen großer Durchbruch das Atmen zusätzlich. In einer Ausführungsform ragt die Zinne zumindest teilweise durch den Durchbruch.

In einer Ausführungsform ist vorgesehen, dass es sich bei dem Mittelteil um ein Schild handelt, das sich im Wesentlichen normal auf den Schnabel erstreckt. In dieser Ausführungsform umfasst das Mundstück also ein zentrales Schild. Dieses soll bei Anwendung der Vorrichtung vorzugsweise im Wesentlichen parallel zur Frontalebene des Patienten ausgerichtet sein und mithin in der Auflageebene der Maske auf das Mundstück liegen. Beispielsweise kann es eine kreisförmige oder ovale Grundgestalt haben.

In einer Ausführungsform ist vorgesehen, dass der Schnabel eine abgeflachte Gestalt hat. Eine abgeflachte Gestalt erlaubt es dem Patienten, den Schnabel mit seinen Lippen komfortabel zu umschließen und bei nur wenig geöffnetem Kiefer auf den Schnabel zu beißen. Die abgeflachte Gestalt leistet einen wesentlichen Beitrag zum Patientenkomfort. In dieser Ausführungsform eignet sich die Vorrichtung insbesondere zur Anwendung bei der Bestrahlung eines Hirntumors.

In einer alternativen Ausführungsform ist vorgesehen, dass die Höhe und die Breite des Schnabels sich ungefähr entsprechen. Durch die bauchigere Gestalt des Schnabels kann eine bestimmte Ober- und Unterkieferpositionierung und eine Fixation der Zunge am Mundboden erreicht werden. In dieser Ausführungsform eignet sich die Vorrichtung insbesondere zur Anwendung bei der Bestrahlung eines HNO-Tumors.

In einer Ausführungsform sind am Schnabel und vorzugsweise im Übergangsbereich zwischen Schnabel und Schild gegenüberliegende Aufnahmevertiefungen vorgesehen. Diese dienen der Aufnahme von Lippen und Zähnen des Patienten.

In einer Ausführungsform ist vorgesehen, dass der Luftkanal eine Bohrung umfasst, die vorzugsweise im Wesentlichen senkrecht auf die Auflageebene der Maske verläuft. Der Luftkanal ermöglicht es dem Patienten, durch den Mund zu atmen, trotzdem er den Schnabel im Mund hat und mit seinen Lippen umschließt.

Die Erfindung betrifft ferner ein Verfahren zur Herstellung einer erfindungsgemäßen Vorrichtung, wobei eine plane und noch nicht ausgeformte thermoplastische Maske erwärmt wird, bis das Maskenmaterial formbar ist, wobei die so erwärmte Maske über den Kopf eines Patienten gezogen wird, der den Schnabel des Mundstücks im Mund hält, sodass die Haltestruktur zum Maskenmaterial hin gerichtet ist, und wobei die Maske dann an einer relativ zum Patienten ortsfesten Halterung fixiert wird, bis sie durch Abkühlung erstarrt.

Die Maske kann im fixierten Zustand aktiv gekühlt werden, bevorzugt ist aber ein einfaches Warten, bis die Maske von selbst unter den Erweichungspunkt des thermoplastischen Materials abkühlt. Die Halterung kann an einem Behandlungstisch einer Anlage zur Durchführung einer Strahlentherapie montiert sein, auf dem auch der Patient positioniert ist. Während dem Überziehen bilden sich die zum Kopf des Patienten und die zur Haltestruktur korrespondierenden Ausformungen bzw. Konturen in der Maske aus, die dann durch Erstarren der Maske darin fixiert werden. Während des Verfahrens und auch im Nachgang erfolgt vorzugsweise keine Verbindung des Maskenmaterials mit dem Mundstück. Das Mundstück und die Maske liegen stets als separate Teile vor, die getrennt und zusammengefügt werden können, in dem das Mundstück in die korrespondierende Kontur der Maske eingesetzt und wieder daraus entnommen wird.

Weiterhin betrifft die Erfindung die Verwendung einer erfindungsgemäßen Vorrichtung zur Immobilisierung des Kopfes eines Patienten in einer Anlage zur Durchführung einer Strahlentherapie. Nach der Herstellung kann die patientenspezifisch geformte Maske bei jeder weiteren Immobilisierung verwendet werden, beispielsweise im Rahmen von Diagnose- oder Therapieschritten. Dabei ist vorgesehen, dass der Patient wieder dasselbe oder ein identisches Mundstück in den Mund nimmt und die seit der erfindungsgemäßen Herstellung aufbewahrte Maske wieder aufgesetzt wird.

Letztlich betrifft die Erfindung auch eine Anlage zur Durchführung einer Strahlentherapie, welche zur Immobilisierung des Kopfes eines Patienten eine erfindungsgemäße Vorrichtung umfasst. In einer Ausführungsform umfasst die Anlage einen Behandlungstisch, an dem die thermoplastische Maske, gegebenenfalls mittels eines Rahmens fixiert ist. Der Behandlungstisch kann eine Basisplatte mit Mitteln zur Fixierung der Maske bzw. des Rahmens aufweisen oder selbst derartige Mittel zur Fixierung umfassen.

Weitere Einzelheiten und Vorteile der Erfindung ergeben sich aus dem nachfolgend anhand der Figuren erklärten Ausführungsbeispiel. In den Figuren zeigen:
- Figur 1:: eine erste Ausführungsform eines Mundstücks einer erfindungsgemäßen Vorrichtung;
- Figur 2:: eine zweite Ausführungsform eines Mundstücks einer erfindungsgemäßen Vorrichtung;
- Figur 3:: Ansichten einer thermoplastischen Maske einer erfindungsgemäßen Vorrichtung; und
- Figur 4:: eine dritte Ausführungsform eines Mundstücks einer erfindungsgemäßen Vorrichtung.

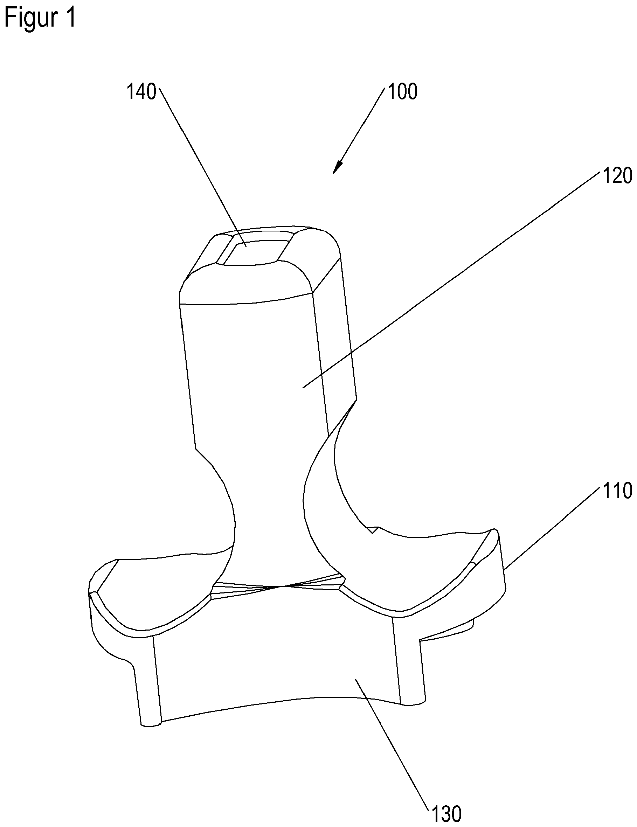

In Figur 1 wird ein Mundstück 100 einer Ausführungsform einer erfindungsgemäßen Vorrichtung in perspektivischer Ansicht gezeigt. Das Mundstück ist insbesondere zur Anwendung bei der Bestrahlung eines Hirntumors geeignet.

Das Mundstück 100 umfasst ein zentrales Schild 110, das bei Anwendung der Vorrichtung im Wesentlichen parallel zur Frontalebene des Patienten liegt. Es hat im gezeigten Beispiel eine kreisförmige Grundgestalt, wobei an den linken und rechten Hochseiten jeweils teilkreisförmige Einbuchtungen vorgesehen sind. Ausgehend von diesem Schild 110 erstreckt sich ein waagrecht abgeflachter Schnabel 120 in orale Richtung. Die Breite des Schnabels 120 entspricht im gezeigten Beispiel in etwa dem Abstand der Scheitelpunkte der Einbuchtungen. Der Schnabel 120 wird bei Anwendung vom Patienten in den Mund genommen. Das Schild 110 dient als Begrenzung und legt die Eindringtiefe des Schnabels 120 in den Mund des Patienten fest, wobei insbesondere ein zu tiefes Eindringen verhindert werden soll.

Im Übergangsbereich zwischen Schnabel 120 und Schild 110 sind gegenüberliegende Aufnahmevertiefungen für Lippen und Zähne vorgesehen. Durch seine spezielle Passform eignet sich das Mundstück 100 auch für zahnlose Patienten.

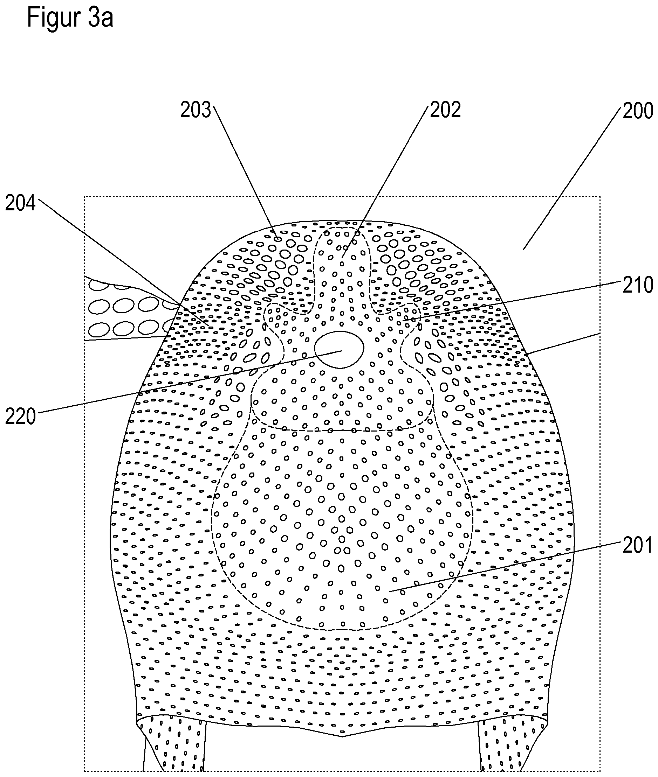

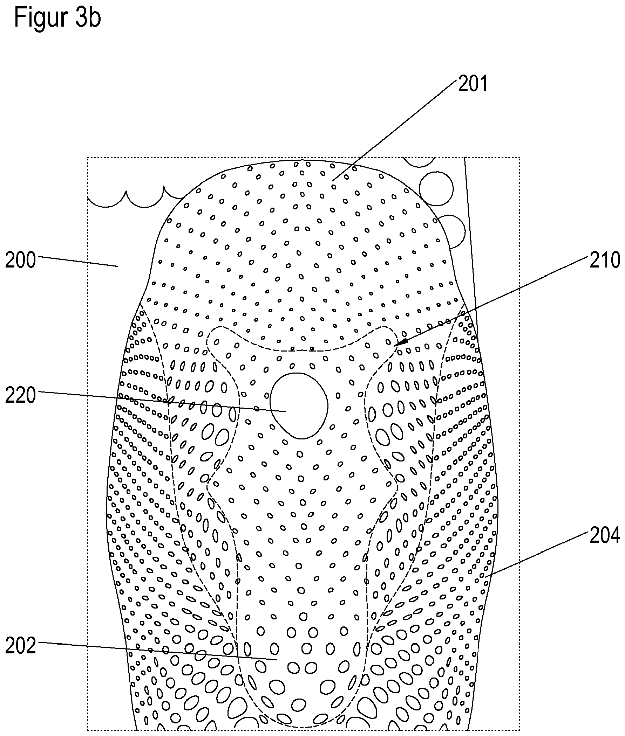

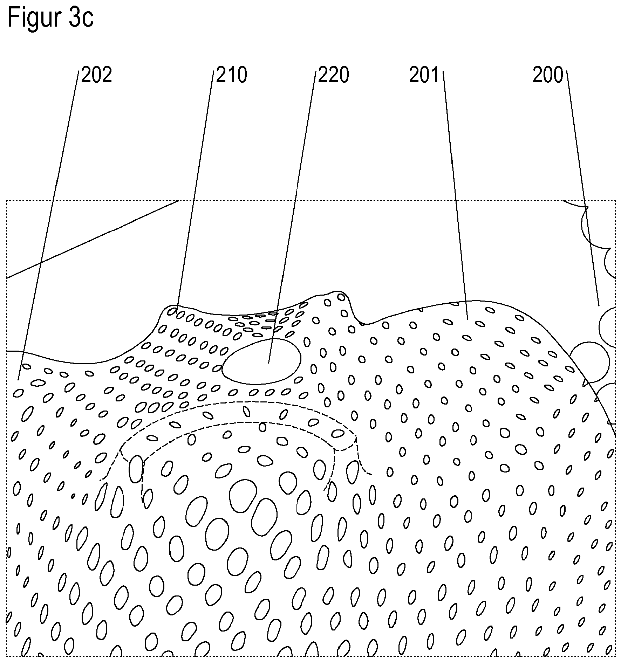

An seiner aboralen Seite ist das Schild 110 mit einer Haltestruktur 130 versehen. Bei der Haltestruktur 130 handelt es sich im gezeigten Beispiel um zwei senkrecht vom Schild abstehende Stege, die dem Kantenverlauf der Einbuchtungen im Schild 110 folgen. Die Kontur dieser Haltestruktur 130 wird bei Anwendung in der Maske 200 (Figur 3) der Vorrichtung abgebildet, um eine genaue und reproduzierbare Position des Mundstücks 100 im Verhältnis zur Maske 200 festlegen zu können.

Das Mundstück 100 umfasst ferner einen Luftkanal 140, der sich vom mundseitigen Ende des Schnabels 120 bis an die aborale Seite des Schildes 110 erstreckt und das Mundstück 100 so von der oralen bis zur aboralen Seite durchdringt. Der Luftkanal 140 ermöglicht es dem Patienten, durch den Mund zu atmen, trotzdem er den Schnabel 120 im Mund hat und mit seinen Lippen umschließt. Bei dem Luftkanal 140 handelt es sich im gezeigten Beispiel um eine einzelne Bohrung.

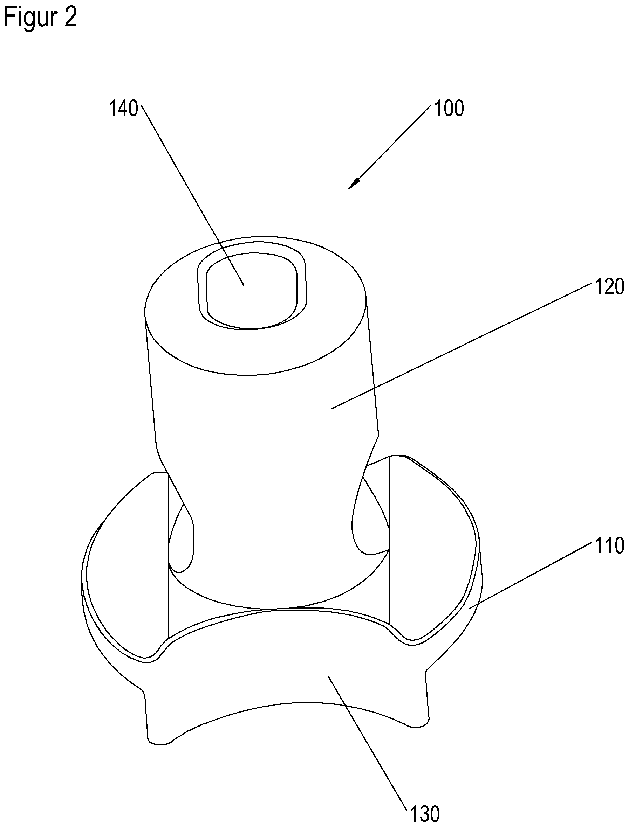

In Figur 2 wird ein Mundstück 100 einer weiteren Ausführungsform einer erfindungsgemäßen Vorrichtung in perspektivischer Ansicht gezeigt. Das Mundstück 100 der Figur 2 ist insbesondere zur Anwendung bei der Bestrahlung eines HNO-Tumors geeignet. Für korrespondierende Teile werden in der Figur 2 identische Bezugszeichen verwendet. Das Mundstück 100 der Figur 2 unterscheidet sich vom Mundstück der Figur 1 durch die bauchigere und im Querschnitt annähernd runde Gestalt des Schnabels 120. Durch diese alternative Gestaltung kann eine bestimmte Ober- und Unterkieferpositionierung und eine Fixation der Zunge am Mundboden erreicht werden.

In Figuren 3a-3c wird eine Maske 200 einer Ausführungsform einer erfindungsgemäßen Vorrichtung aus mehreren Perspektiven gezeigt. Figur 3a zeigt die Maske 200 - mit Bezugnahme auf den Patienten - von schräg unten, Figur 3b von schräg oben und Figur 3c von schräg rechts.

Die Maske 200 wird durch einen blattförmigen Bogen aus einem thermoplastischen Kunststoffmaterial dargestellt, der entsprechend der Kontur des Patientenkopfes ausgeformt ist. In den Figuren sind unter anderem Konturen entsprechend dem Kinn (Bezugszeichen 201), der Nase (Bezugszeichen 202), der Stirn (Bezugszeichen 203) und der Backen (Bezugszeichen 204) erkennbar. Der Bogen ist engmaschig mit kleinen Löchern durchsetzt, sodass er eine nahezu netzförmige Gestalt hat. Dies erhöht den Komfort des Patienten und ermöglicht es ihm, seine Umgebung wahrzunehmen. Ferner wird auch die Ausformung des originär ebenen Bogens durch die Löcher erleichtert. Der die Maske 200 darstellende Bogen ist am hinteren Ende, wie in Figur 3a ansatzweise erkennbar, in einen Maskenrahmen 300 eingefasst, der auf einem nicht dargestellten Behandlungstisch einer Anlage zur Durchführung einer Strahlentherapie fixiert werden kann.

In demjenigen Bereich des Bogens, der den Mundbereich des Patienten überdeckt, ist eine Mundstückkontur 210 erkennbar, welche der Haltestruktur 130 des Mundstücks 100 (Figuren 1 oder 2) entspricht. Anhand dieser Mundstückkontur 210 wird eine genaue und reproduzierbare Position des Mundstücks 100 im Verhältnis zur Maske 200 festgelegt. Im gezeigten Beispiel hat die Mundstückkontur 210 eine X-ähnliche Form, was sich als Konsequent aus der Form der Haltestruktur 130 des Mundstücks 100 ergibt. Die vier Haltepunkte an den Eckkanten begünstigen dabei eine drehsichere Festlegung.

An einer Stelle der Maske 200, die bei Anwendung direkt über dem Mund des Patienten liegt, ist ein Durchbruch eingearbeitet. Er liegt entsprechend etwa im Zentrum der Mundstückkontur 210. Im gezeigten Beispiel ist der Durchbruch 220 etwa kreisförmig. Aufgrund des Durchbruchs 220 liegt der Luftkanal 140 des Mundstücks 100 (Figuren 1 oder 2) bei Anwendung der Vorrichtung frei und die Atmung des Patienten wird nicht erschwert.

Anhand der gezeigten Vorrichtung kann eine Immobilisierung des Kopfes eines Patienten in einer Anlage zur Durchführung einer Strahlentherapie erfolgen.

Bei der initialen Anpassung nimmt der Patient zunächst den Schnabel 120 des Mundstücks 100 so in den Mund, dass die Vertiefungen zwischen Zähnen und Lippen eingefasst sind. Anschließend wird, wie grundsätzlich aus dem Stand der Technik bekannt, die Maske 200 mit erwärmtem Maskenmaterial über den Kopf des Patienten gezogen, sodass im originär ebenen Maskenmaterial die Kontur des Patientenkopfes ausgeformt wird. Im Unterscheid zum Stand der Technik wird dabei aber nicht nur die Kontur des Patientenkopfes, sondern zusätzlich auch die Haltestruktur 130 des Mundstücks 100 als Mundstückkontur 220 im Maskenmaterial abgebildet. Sodann härtet das Maskenmaterial durch Abkühlung aus. Nachdem das Maskenmaterial ausgehärtet ist, wird die Maske 200 vom Patienten abgenommen und das Mundstück 100 vom Patienten entfernt. Der Durchbruch 220 wird nachträglich ausgeschnitten. Die Maske 200 mit dem ausgehärteten und patientenspezifisch ausgeformten Maskenmaterial wird für weitere Immobilisierungen gelagert. Das Mundstück 100 kann wahlweise für die Wiederverwendung gereinigt oder verworfen werden.

Bei jeder weiteren Immobilisierung, beispielsweise im Rahmen von Diagnose- oder Therapieschritten nimmt der Patient wieder dasselbe oder ein identisches Mundstück 100 in den Mund und die seit der oben beschriebenen, initialen Anpassung aufbewahrte Maske 200 mit dem ausgehärteten und patientenspezifisch ausgeformten Maskenmaterial wird wieder aufgesetzt.

Anhand dieses neuartigen Systems kann eine hohe Positioniergenauigkeit der Maske 200 am Kopf des Patienten erreicht werden und Kopfbewegungen und insbesondere Kieferbewegungen können weitgehend vermieden werden. Zugleich wird die Atmung des Patienten kaum beeinträchtigt und der Patientenkomfort wird im Vergleich zu bestehenden Lösungen verbessert.

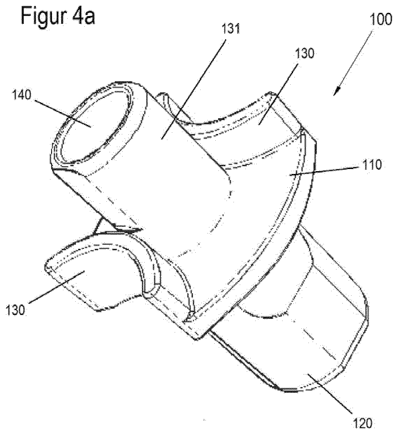

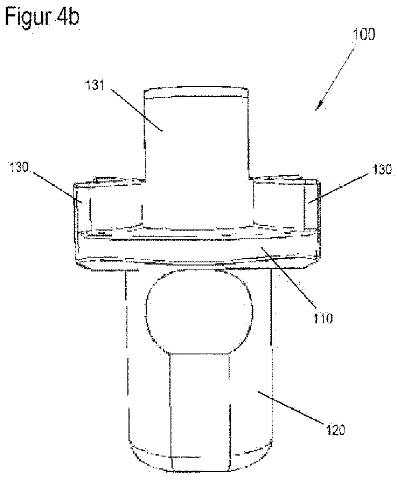

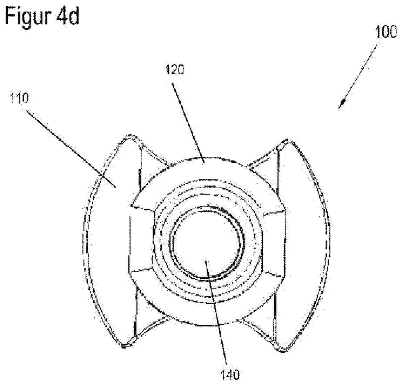

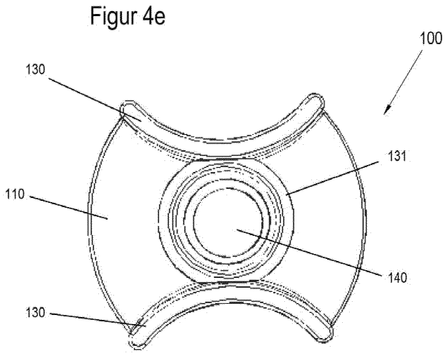

Figuren 4a-4e zeigen letztlich eine weitere Ausführungsform eines Mundstücks 100 einer erfindungsgemäßen Vorrichtung, und zwar in perspektivischer Darstellung (Figur 4a), in Seitenansichten (Figuren 4b-c) sowie als Draufsichten von der oralen (Figur 4d) und der aboralen (Figur 4e) Seite.

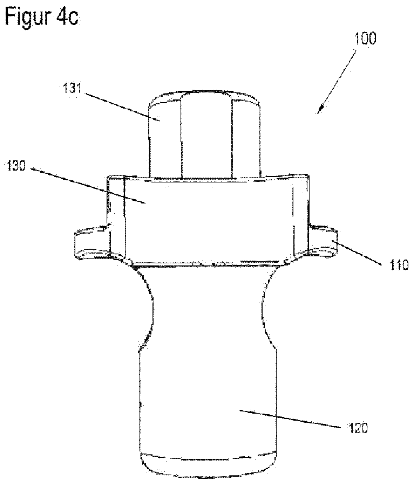

Das Mundstück 100 der Ausführungsform gemäß Figur 4 ist dem Mundstück der Ausführungsform gemäß Figur 2 grundsätzlich sehr ähnlich. Das einzige Unterscheidungsmerkmal ist das zusätzliche die zusätzliche aborale Haltestruktur in Form einer Zinne 131, die in der Achse des Schnabels vertikal vom Mittelteil 110 absteht und die Stege 130 in aborale Richtung deutlich überragt. Der Luftkanal 140 setzt sich in dieser Ausführungsform bis an das aborale Ende der Zinne 131 fort und endet entsprechend nicht bereits an der aboralen Seite des Schildes 110.

Durch die Zinne 131 kann die Fixierung der Maske 200 am Mundstück 100 verbessert werden. Ferner wird durch das Abheben der Austrittsöffnung des Luftkanals 140 vom Schild 110 im Falle eines Erbrechens des Patienten vermieden, dass das Erbrochene wieder zurück in den Luftkanal 140 gelangen kann. Die Zinne 131 kann bei Aufsetzen der Maske 200 unter Umständen zumindest teilweise durch den Durchbruch 220 ragen, wobei jedoch bevorzugt ist, dass das Maskenmaterial so ausgeformt wird, dass der Durchbruch 220 auf der Spitze der Zinne 131 aufliegt. bei Aufsetzen der Maske 200 unter Umständen zumindest teilweise durch den Durchbruch 220 ragen, wobei jedoch bevorzugt ist, dass das Maskenmaterial so ausgeformt wird, dass der Durchbruch 220 auf der Spitze der Zinne 131 aufliegt.

## Patentansprüche

1. Vorrichtung zur Immobilisierung des Kopfes eines Patienten in einer Anlage zur Durchführung einer Strahlentherapie, wobei die Vorrichtung eine entsprechend der Kontur des Patientenkopfes ausgeformte thermoplastische Maske (200) und ein Mundstück (100) umfasst, wobei das Mundstück (100) ein Mittelteil (110) aufweist, von dem in orale Richtung ein Schnabel (120) und in aborale Richtung eine Haltestruktur (130) abstehen, und wobei ein Luftkanal (140) vorgesehen ist, der das Mundstück (100) vom mundseitigen Ende des Schnabels (120) bis an die aborale Seite des Mittelteils (110) durchdringt,
**dadurch gekennzeichnet,**
**dass** ein Bereich der Maske (200) entsprechend der Kontur der Haltestruktur (130) ausgeformt ist.

2. Vorrichtung nach Anspruch 1 wobei das Mundstück (100) nicht mit der Maske (200) verbunden ist und mit seiner Haltestruktur (130) lose in der entsprechenden Ausformung (210) der Maske (200) sitzt.

3. Vorrichtung nach einem der vorhergehenden Ansprüche, wobei die Haltestruktur (130) wenigstens zwei von dem Mittelteil (110) abstehende Strukturelemente aufweist und/oder dass ein oder mehrere Strukturelemente der Haltestruktur (130) eine in der Auflageebene der Maske (200) ausgedehnte Gestalt haben.

4. Vorrichtung nach Anspruch 3, wobei die Strukturelemente eine in der Achse des Schnabels (120) von dem Mittelteil (110) abstehende Zinne (131) umfassen, wobei sich der Luftkanal (140) bis zum aboralen Ende dieser Zinne (131) fortsetzt.

5. Vorrichtung nach einem der vorhergehenden Ansprüche, wobei in demjenigen Bereich (210) der Maske (200), der entsprechend der Kontur der Haltestruktur (130) ausgeformt ist, ein Durchbruch (220) eingearbeitet ist.

6. Vorrichtung nach einem der vorhergehenden Ansprüche, wobei es sich bei dem Mittelteil (110) um ein Schild handelt, das sich im Wesentlichen normal auf den Schnabel (120) erstreckt.

7. Vorrichtung nach einem der vorhergehenden Ansprüche, wobei am Schnabel (120) und vorzugsweise im Übergangsbereich zwischen Schnabel (120) und Schild gegenüberliegende Aufnahmevertiefungen (115) vorgesehen sind.

8. Vorrichtung nach einem der vorhergehenden Ansprüche, wobei der Luftkanal (140) eine Bohrung umfasst, die vorzugsweise im Wesentlichen senkrecht auf die Auflageebene der Maske (200) verläuft.

9. Verfahren zur Herstellung einer Vorrichtung nach einem der vorhergehenden Ansprüche, wobei eine plane und noch nicht ausgeformte thermoplastische Maske (200) erwärmt wird, bis das Maskenmaterial formbar ist, dass die so erwärmte Maske (200) über den Kopf eines Patienten gezogen wird, der den Schnabel (120) des Mundstücks (100) im Mund hält, sodass die Haltestruktur (130) zum Maskenmaterial hin gerichtet ist, und dass die Maske (200) dann an einer relativ zum Patienten ortsfesten Halterung fixiert wird, bis sie durch Abkühlung erstarrt.

10. Verwendung einer Vorrichtung nach einem der Ansprüche 1 bis 8 zur Immobilisierung des Kopfes eines Patienten in einer Anlage zur Durchführung einer Strahlentherapie.

11. Anlage zur Durchführung einer Strahlentherapie, welche zur Immobilisierung des Kopfes eines Patienten eine Vorrichtung nach einem der Ansprüche 1 bis 8 umfasst.

## Claims

1. A device for immobilising the head of a patient in an installation for carrying out radiotherapy, wherein the device comprises a thermoplastic mask (200) shaped according to the contour of the patient's head and a mouthpiece (100), wherein the mouthpiece (100) has a central part (110) from which a spout (120) protrudes in the oral direction and from which a holding structure (130) protrudes in the aboral direction, and wherein an air channel (140) is provided which passes through the mouthpiece (100) from the mouth-side end of the spout (120) to the aboral side of the central part (110),
**characterised in that**
a region of the mask (200) is shaped according to the contour of the holding structure (130).

2. The device according to claim 1, wherein the mouthpiece (100) is not connected to the mask (200) and sits with its holding structure (130) loosely in the corresponding shaping (210) in the mask (200).

3. The device according to one of the preceding claims, wherein the holding structure (130) has at least two structural elements protruding from the central part (110) and/or in that one or more structural elements of the holding structure (130) have an elongated shape in the contact plane of the mask (200).

4. The device according to claim 3, wherein the structural elements comprise a merlon (131) protruding from the central part (110) in the axis of the spout (120), wherein the air channel (140) continues to the aboral end of this merlon (131).

5. The device according to one of the preceding claims, wherein an aperture (220) is incorporated in the region (210) of the mask (200) that is shaped according to the contour of the holding structure (130).

6. The device according to one of the preceding claims, wherein the central part (110) is a shield, which extends in a manner substantially normal to the spout (120).

7. The device according to one of the preceding claims, wherein accommodating recesses (115) are provided opposite one another on the spout (120) and preferably in the transition region between spout (120) and shield.

8. The device according to one of the preceding claims, wherein the air channel (140) comprises a bore, which preferably runs substantially perpendicular to the contact plane of the mask (200).

9. A method for producing a device according to one of the preceding claims, wherein a flat and not yet shaped thermoplastic mask (200) is heated until the mask material is formable, in that the mask (200) that has been thus heated is stretched over the head of a patient, who holds the spout (120) of the mouthpiece (100) in his mouth such that the holding structure (130) is directed towards the mask material, and in that the mask (200) is then secured to a holder that is in a fixed position relative to the patient until it sets by cooling.

10. Use of a device according to one of claims 1 to 8 for immobilising the head of a patient in an installation for carrying out radiotherapy.

11. An installation for carrying out radiotherapy which, in order to immobilise the head of a patient, comprises a device according to one of claims 1 to 8.

## Revendications

1. Dispositif d'immobilisation de la tête d'un patient dans une installation destinée à réaliser une radiothérapie, le dispositif comprenant un masque (200) thermoplastique façonné conformément au contour de la tête du patient et un élément buccal (100), l'élément buccal (100) présentant une partie médiane (110) depuis laquelle dépassent, dans la direction orale, un bec (120) et, dans la direction aborale, une structure de maintien (130), et un canal d'air (140) étant prévu, lequel traverse l'élément buccal (100) de l'extrémité côté bouche du bec (120) jusqu'au côté aboral de la partie médiane (110),
**caractérisé en ce que**
une zone du masque (200) est façonnée conformément au contour de la structure de maintien (130).

2. Dispositif selon la revendication 1, dans lequel l'élément buccal (100) n'est pas relié au masque (200) et se loge avec sa structure de maintien (130) libre dans la forme (210) correspondante du masque (200).

3. Dispositif selon l'une des revendications précédentes, dans lequel la structure de maintien (130) présente au moins deux éléments de structure dépassant de la partie médiane (110) et/ou en ce qu'un ou plusieurs éléments de structure appartenant à la structure de maintien (130) ont une forme étendue dans le plan de pose du masque (200).

4. Dispositif selon la revendication 3, dans lequel les éléments de structure comprennent un pinacle (131) dépassant de la partie médiane (110) dans l'axe du bec (120), le canal d'air (140) s'étendant jusqu'à l'extrémité aborale dudit pinacle (131).

5. Dispositif selon l'une des revendications précédentes, dans lequel une ouverture (220) est réalisée dans la zone (210) du masque (200) qui est façonnée conformément au contour de la structure de maintien (130).

6. Dispositif selon l'une des revendications précédentes, dans lequel la partie médiane (110) est un écran qui s'étend sensiblement de manière perpendiculaire par rapport au bec (120).

7. Dispositif selon l'une des revendications précédentes, dans lequel des creux de réception (115) opposés sont prévus sur le bec (120) et de préférence dans la zone de transition entre le bec (120) et l'écran.

8. Dispositif selon l'une des revendications précédentes, dans lequel le canal d'air (140) présente un trou qui s'étend de préférence sensiblement perpendiculairement au plan de pose du masque (200).

9. Procédé de fabrication d'un dispositif selon l'une des revendications précédentes,
dans lequel un masque (200) thermoplastique plan et pas encore façonné est chauffé jusqu'à ce que le matériau du masque puisse être façonné, en ce que le masque (200) ainsi chauffé est enfilé sur la tête d'un patient qui tient le bec (120) de l'élément buccal (100) dans la bouche, de telle sorte que la structure de maintien (130) est orientée vers le matériau du masque et en ce que le masque (200) est ensuite fixé sur un support fixe par rapport au patient jusqu'à ce qu'il se solidifie par refroidissement.

10. Utilisation d'un dispositif selon l'une des revendications 1 à 8 pour immobiliser la tête d'un patient dans une installation destinée à réaliser une radiothérapie.

11. Installation destinée à réaliser une radiothérapie, qui comprend, pour immobiliser la tête d'un patient, un dispositif selon l'une des revendications 1 à 8.
